# EUROPÄISCHE PATENTSCHRIFT

(11) **EP 3 405 195 B1**
(45) Veröffentlichungstag und Bekanntmachung des Hinweises auf die Patenterteilung: **12.03.2025**
(21) Anmeldenummer: 17703665.4
(22) Anmeldetag: 20.01.2017
(51) Int. Cl.: A61K 31/4545, A61K 9/16, A61K 9/20

(54) **VERFAHREN ZUR HERSTELLUNG EINES APIXABAN-GRANULATES**
METHOD FOR PRODUCING AN APIXABAN GRANULATE
PROCEDE DE FABRICATION D'UN GRANULE D'APIXABAN

(30) Priorität: 22.01.2016 EP 16000150
(43) Veröffentlichungstag der Anmeldung: 28.11.2018
(73) Patentinhaber: STADA Arzneimittel AG, 61118 Bad Vilbel (DE)
(72) Erfinder: SCHÖNBORN, Jessica, 61118 Bad Vilbel (DE)
(74) Vertreter: Kernebeck, Thomas
(86) Internationale Anmeldenummer: PCT/EP2017/051162
(87) Internationale Veröffentlichungsnummer: WO 2017/125535

(56) Entgegenhaltungen:
- EP-A1- 2 554 159
- EP-A1- 2 907 507
- WO-A2-2011/106478
- CN-A- 104 490 834
- US-A1- 2015 064 252
- US-A1- 2015 272 891
- BASF: "Pharma Ingredients & Services = Registered trademark of BASF group Soluplus Technical Information Supersedes issue dated May 2010 03_090801e-04/Page 1 of 8", 31 July 2010 (2010-07-31), XP055555966, Retrieved from the Internet <URL:https://industries.basf.com/en/documentDownload.8805242743253.Soluplus %20-%20Technical%20Information.pdf> [retrieved on 20190213]

## Beschreibung

Die vorliegende Erfindung betrifft ein Verfahren zur Herstellung eines pharmazeutischen Granulats, enthaltend als Wirkstoff Apixaban oder ein Salz davon.

Apixaban ist der internationale Freiname (INN (*international nonproprietary name))* für 1-(4-Methoxyphenyl)-7-oxo-6-[4-(2-oxopiperidin-1-yl)phenyl]-4,5,6,7-tetrahydro-1H-pyrazolo[3,4-c]pyridin-3-carbamid, das die folgende Strukturformel aufweist: 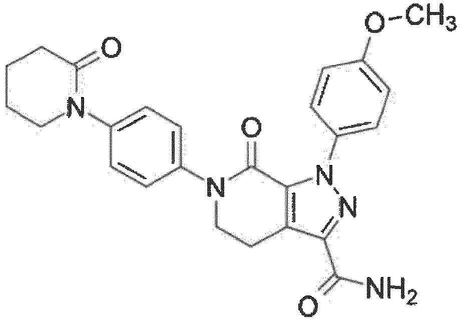 Apixaban ist ein Arzneistoff aus der Gruppe der Antikoagulanzien, der für die
- Prophylaxe von Schlaganfällen und systemischen Embolien bei erwachsenen Patienten mit nicht-valvulärem Vorhofflimmern und einem oder mehreren Risikofaktoren, wie Schlaganfall oder TIA (transitorischer ischämischer Attacke) in der Anamnese, Alter ≥ 75 Jahren, Hypertonie, Diabetes mellitus, symptomatische Herzinsuffizienz (NYHA Klasse ≥ II);
- Behandlung von tiefen Venenthrombosen (TVT) und Lungenembolien (LE) sowie Prophylaxe von rezidivierenden TVT und LE bei Erwachsenen;
- Thromboembolieprophylaxe nach Knie- und Hüftgelenksersatz;
zugelassen ist. Die Wirkung von Apixaban beruht dabei auf der selektiven Hemmung des an der Blutgerinnung beteiligten Enzyms Faktor Xa.

Apixaban wird unter der Markenbezeichnung Eliquis^{®} in Form von oralen Filmtabletten in den Wirkstärken 2,5 mg und 5 mg Apixaban vertrieben. Apixaban ist in Eliquis^{®} in Form der freien Base enthalten. Ausweislich des Bewertungsberichts der Europäischen Arzneimittelbehörde (European Medicines Agency (EMA)) zu Eliquis^{®} vom 20. Juni 2011 (vgl. unter Punkt 2 "scientific discussion") werden besagte orale Filmtabletten über den Zwischenschritt eines Trockengranulierungsverfahrens hergestellt. Es wird ausgeführt, dass mittels eines Apixaban-Trockengranulats Tabletten herstellbar sind, die im Hinblick auf eine unverzögerte Wirkstofffreisetzung zwar ausreichend konsistente Auflösungsraten zeigen, allerdings muss hierzu der Wirkstoff in einer bestimmten Partikelgrößenverteilung eingesetzt werden.

EP 2 907 507 A1 offenbart Apixaban-Tabletten, die neben einem niedrig-viskosen Polymer ein Tensid enthalten. So kommt Natriumlaurylsulfat als Tensid und Povidon (Polyvinlypyrrolidon) als niedrigviskoses Polymer zum Einsatz.

Aufgabe der vorliegenden Erfindung ist es daher, ein Verfahren zur Herstellung eines pharmazeutischen Granulats, enthaltend als Wirkstoff Apixaban oder ein Salz davon, bereitzustellen, in welchem der Wirkstoff weitgehend unabhängig von seiner Partikelgrößenverteilung eingesetzt werden kann, wobei mittels des resultierenden Granulats aber dennoch Darreichungsformen hergestellt werden können, die ausreichend konsistente Wirkstoff-Auflösungsraten für eine unverzögerte Wirkstofffreisetzung zeigen.

Die Aufgabe wird gelöst durch ein Verfahren der eingangs genannten Art, umfassend die Schritte des
a) Bereitstellens eines Lösungsmittels;
b) Zusammenführens des Lösungsmittels mit dem Wirkstoff unter Erhalt einer Granulierflüssigkeit;
c) Feuchtgranulierens eines pulverförmigen Materials mittels der Granulierflüssigkeit,
wobei in dem Lösungsmittel eine amphiphile Substanz enthalten ist und die amphiphile Substanz ein Polyvinylcaprolactampolyvinylacetat-polyethylenglykol- pfropfcopolymer ist oder ausgewählt ist aus der Gruppe bestehend aus Caprylocaproylmacrogol-8-glycerid, Lauroyl-macrogol-32-glycerid, Stearoylmacrogol-32-glycerid, Oleoyl-macrogol-6-glycerid, Linoleoylmacrogol-6-glycerid und Lauroyl-macrogol-6-glycerid, und der Wirkstoff in dem Lösungsmittel gelöst, gelöst und dispergiert, gelöst und suspendiert oder gelöst, dispergiert und suspendiert enthalten ist.

Überraschenderweise wurde festgestellt, dass in dem erfindungsgemäßen Verfahren der Wirkstoff weitgehend unabhängig von seiner Partikelgrößenverteilung eingesetzt werden kann, aber dennoch Granulate resultieren, mittels derer Darreichungsformen herstellbar sind, die ausreichend konsistente Wirkstoff-Auflösungsraten für eine unverzögerte Wirkstofffreisetzung zeigen.

Da in dem erfindungsgemäßen Verfahren der Wirkstoff weitgehend unabhängig von seiner Partikelgrößenverteilung eingesetzt werden kann, ist das erfindungsgemäße Verfahren verhältnismäßig kostengünstig durchführbar. Auch eine etwaige Nachvermahlung agglomerierter Wirkstoffpartikel einer ehemals definierten Partikelgrößenverteilung ist mit Blick auf das erfindungsgemäße Verfahren nicht mehr notwendig.

Darüber hinaus wurde vorteilhafterweise festgestellt, dass sich das mittels des erfindungsgemäßen Verfahrens erhältliche Granulat sowie daraus hergestellte Darreichungsformen durch eine verhältnismäßig hohe Gleichförmigkeit des Wirkstoffgehalts gemäß Europäischem Arzneibuch, 8. Ausgabe, 4. Nachtrag, Kapitel 2.9.6 und 2.9.40 auszeichnen.

In dem erfindungsgemäßen Verfahren kommt ein Lösungsmittel zum Einsatz. Erfindungsgemäß bevorzugt kann dabei als Lösungsmittel im Prinzip jedes Lösungsmittel eingesetzt werden, in dem Apixaban bzw. das pharmazeutisch annehmbare Salz davon löslich ist und mittels dem eine Feuchtgranulation des pulverförmigen Materials, welches in der Regel aus einem oder mehreren pharmazeutisch annehmbaren Hilfsstoffen wie etwa mikrokristalline Cellulose und/oder Lactose gebildet ist, durchgeführt werden kann. Erfindungsgemäß bevorzugte Lösungsmittel umfassen Dimethylsulfoxid (DMSO), Dimethylformamid (DMF), Ethanol, Isopropanol oder Wasser oder Mischungen von zwei oder mehr der vorgenannten, wobei zur Verbesserung der Löslichkeit des Apixabans bzw. des pharmazeutisch annehmbaren Salzes davon das Lösungsmittel zusätzlich ein oder mehrere Solubilisierungsmittel umfassen kann.

In dem erfindungsgemäßen Verfahren wird das Lösungsmittel mit dem Wirkstoff zusammengeführt, wobei zumindest ein Teil des Wirkstoffs in dem Lösungsmittel unter Erhalt einer Granulierflüssigkeit gelöst wird. Das bedeutet, dass der Wirkstoff in dem Lösungsmittel löslich ist und dass er bei gegebenen Bedingungen vorzugsweise auch so vollständig wie möglich gelöst wird. Im Hinblick auf die oben genannten technischen Vorteile der vorliegenden Erfindung ist es grundsätzlich bevorzugt, wenn die Löslichkeit des Wirkstoffs in dem Lösungsmittel möglichst hoch ist. Es ist jedoch ausreichend und erfindungsgemäß bevorzugt, wenn die Löslichkeit des Wirkstoffs in dem Lösungsmittel bei einer Temperatur von 50 °C größer als 0,01 mg/ml ist, weiter bevorzugt größer als 0,1 mg/ml, mehr bevorzugt größer als 0,2 mg/ml und noch mehr bevorzugt größer als 0,5 mg/ml.

Das in dem erfindungsgemäßen Verfahren einzusetzende pulverförmige Material kann im Prinzip jeglicher Natur sein, allerdings muss es zu einem Feuchtgranulat verarbeitet werden können. Als Beispiele für pulverförmige Materialien seien übliche pharmazeutische Hilfsstoffe wie etwa mikrokristalline Cellulose, Lactose, Cellactose^{®}, Crosscarmellose Natrium, Natrium-Carboxymethylstärke, Polyvinylpyrrolidon, Siliciumdioxid, Methylcellulose und Ethylcellulose sowie auch Mischungen von zwei oder mehr der vorgenannten Hilfsstoffe genannt.

Entsprechend dem erfindungsgemäßen Verfahren ist es vorgesehen, dass in dem Lösungsmittel eine amphiphile Substanz vorzugsweise gelöst enthalten ist. Mittels amphiphiler Substanzen kann die Löslichkeit des Wirkstoffs in dem Lösungsmittel verbessert werden. Amphiphile Substanzen sind im Stand der Technik bekannt. Dabei handelt es sich um Moleküle, die sowohl hydrophile als auch hydrophobe Bereiche aufweisen und entsprechend sowohl in polaren Lösungsmitteln als auch in unpolaren Lösungsmitteln verhältnismäßig gut löslich sind.

Gemäß einer bevorzugten Ausführungsform des erfindungsgemäßen Verfahrens ist es vorgesehen, dass das Lösungsmittel Wasser ist oder umfasst. Im Vergleich zu anderen Lösungsmitteln ist Wasser physiologisch unbedenklich. Da das Lösungsmittel nicht zu 100 % aus dem aus dem erfindungsgemäßen Verfahren resultierenden Granulat entfernt werden kann, zeichnet sich bei dem Einsatz von Wasser das resultierende Granulat durch eine erhöhte physiologische Toleranz aus, die im Allgemeinen umso höher ist, je größer der Anteil des Lösungsmittels ist, der von Wasser gebildet wird.

In dem erfindungsgemäßen Verfahren ist es vorgesehen, dass der Wirkstoff in dem Lösungsmittel gelöst, gelöst und dispergiert, gelöst und suspendiert oder gelöst, dispergiert und suspendiert enthalten ist. Wie bereits vorstehend ausgeführt wurde, wird in dem erfindungsgemäßen Verfahren das Lösungsmittel mit dem Wirkstoff zusammengeführt, wobei vzumindest ein Teil des Wirkstoffs in dem Lösungsmittel gelöst wird. Dabei wird der Wirkstoff in dem Lösungsmittel vorzugszugsweise so vollständig wie möglich gelöst, d.h. gegebenenfalls bis zur Löslichkeitsgrenze. Sollte darüber hinaus beispielsweise im Hinblick auf verfahrenstechnische Aspekte der Feuchtgranulierung die Menge an Wirkstoff pro Volumeneinheit Lösungsmittel höher als die maximale Löslichkeit gewünscht sein, so kann der Wirkstoff neben in Lösung auch in dispergierter und/oder suspendierter Form in dem Lösungsmittel vorliegen.

Entsprechend dem erfindungsgemäßen Verfahren ist es vorgesehen, dass der Wirkstoff Apixaban ist. Grundsätzlich kann der Wirkstoff in dem erfindungsgemäßen Verfahren in Form der freien Apixaban-Base oder in Form eines pharmazeutisch annehmbaren Salzes davon eingesetzt werden. Im Hinblick auf die Stabilität des Wirkstoffs in dem resultierenden Granulat ist es allerdings bevorzugt, wenn in dem erfindungsgemäßen Verfahren der Wirkstoff in Form der freien Apixaban-Base eingesetzt wird.

Gemäß einer weiter bevorzugten Ausführungsform des erfindungsgemäßen Verfahrens ist es vorgesehen, dass die amphiphile Substanz eine nicht-ionische Substanz ist. Es konnte festgestellt werden, dass insbesondere nicht-ionische amphiphile Substanzen eine verhältnismäßig leichte Prozessführung im Verfahrensschritt der Feuchtgranulation bewirken können.

Eine amphiphile Substanz kann ausgewählt sein aus der Gruppe bestehend aus Polymeren, Tensiden, Monoglyceriden, Diglyceriden, Triglyceriden und Phospholipiden. Amphiphile Polymere, Tenside, Monoglyceride, Diglyceride, Triglyceride und Phospholipide können eine ausreichende Solubilisierung des Wirkstoffs in dem Lösungsmittel, insbesondere in Wasser, bewirken.

Eine amphiphile Substanz kann ein Pfropfcopolymer, ein Blockcopolymer oder ein Propf- und Blockcopolymer sein. Die besagten Polymere können eine ausreichende Solubilisierung des Wirkstoffs in dem Lösungsmittel bewirken und ferner eine gute Verarbeitbarkeit der Granulierflüssigkeit bei der Feuchtgranulation gewährleisten.

Gemäß dem erfindungsgemäßen Verfahrens ist es vorgesehen, dass die amphiphile Substanz beispielsweise ein Polyvinylcaprolactampolyvinylacetat-polyethylenglykol-pfropfcopolymer ist. Das besagte Polymer kann eine ausreichende Solubilisierung des Wirkstoffs in dem Lösungsmittel bewirken und zeigt im Hinblick auf eine unverzögerte Wirkstofffreisetzung ausreichend konsistente Auflösungsraten des Wirkstoffs. In diesem Zusammenhang ist das Polyvinylcaprolactam-polyvinylacetatpolyethylenglykol-pfropfcopolymer Soluplus^{®} der BASF AG mit einem mittleren Molekulargewicht von 90000 bis 140000 g/mol erfindungsgemäß besonders bevorzugt.

Eine amphiphile Substanz kann ausgewählt sein aus der Gruppe bestehend aus Polyoxyethylenfettsäureestern, Polyoxyethylenfettsäureethern und Polyethylenglykolfettsäurestern. Die genannten amphiphilen Substanzen können eine ausreichende Solubilisierung des Wirkstoffs in dem Lösungsmittel bewirken und ferner eine gute Verarbeitbarkeit der Granulierflüssigkeit bei der Feuchtgranulation gewährleisten.

Entsprechend dem erfindungsgemäßen Verfahren ist es vorgesehen, dass die amphiphile Substanz weiter ausgewählt ist aus der Gruppe bestehend aus Caprylocaproyl-macrogol-8-glycerid, Lauroyl-macrogol-32-glycerid, Stearoyl-macrogol-32-glycerid, Oleoyl-macrogol-6-glycerid, Linoleoyl-macrogol-6-glycerid und Lauroyl-macrogol-6-glycerid, vorzugsweise bestehend aus Lauroylmacrogol-32-glycerid. Die genannten amphiphilen Substanzen können eine ausreichende Solubilisierung des Wirkstoffs in dem Lösungsmittel bewirken und zeigen im Hinblick auf eine unverzögerte Wirkstofffreisetzung sehr konsistente Auflösungsraten des Wirkstoffs. In diesem Zusammenhang ist es erfindungsgemäß besonders bevorzugt, wenn das Lauroyl-macrogol-32-glycerid in Form des Produktes Gelucire^{®} 44/14 der Firma Gattefossé eingesetzt wird. Gelucire^{®} 44/14 ist eine Mischung von PEG-Estern, d.h. Lauroyl-macrogol-32-glycerid, einer kleinen Glycerid-Fraktion und freiem PEG.

Gemäß einer weiter bevorzugten Ausführungsform des erfindungsgemäßen Verfahrens ist es vorgesehen, dass die amphiphile Substanz einen HLB-Wert von 9 bis 18 aufweist, vorzugsweise einen HLB-Wert von 9 bis 12. Amphiphile Substanzen mit HLB(hydrophilic-lipophilic-balance)-Werten in den genannten Bereichen können eine ausreichende Solubilisierung des Wirkstoffs in dem Lösungsmittel bewirken. HLB-Werte für amphiphile Substanzen werden nach anerkannten Methoden berechnet und sind im Stand der Technik bekannt.

Nach einer weiter bevorzugten Ausführungsform des erfindungsgemäßen Verfahrens ist es vorgesehen, dass in der Granulierflüssigkeit Wirkstoff und Lösungsmittel in einem Gewichtsverhältnis von 1:100 bis 1:2500 vorliegen, weiter bevorzugt in einem Gewichtsverhältnis von 1:200 bis 1:1000, mehr bevorzugt in einem Gewichtsverhältnis von 1:200 bis 1:600. Dadurch wird eine gute Verarbeitbarkeit der Granulierflüssigkeit im Feuchtgranulierungsschritt gewährleistet.

Entsprechend einer weiter bevorzugten Ausführungsform des erfindungsgemäßen Verfahrens ist es vorgesehen, dass in der Granulierflüssigkeit Wirkstoff und Lösungsmittel in einem Gewichtsverhältnis von 1:10 bis 1:200 vorliegen, weiter bevorzugt in einem Gewichtsverhältnis von 1:10 bis 1:100, mehr bevorzugt in einem Gewichtsverhältnis von 1:15 bis 1:50. Dadurch kann ebenfalls eine gute Verarbeitbarkeit der Granulierflüssigkeit im Feuchtgranulierungsschritt gewährleistet werden.

Entsprechend einer weiter bevorzugten Ausführungsform des erfindungsgemäßen Verfahrens ist es vorgesehen, dass in der Granulierflüssigkeit Wirkstoff und amphiphile Substanz in einem Gewichtsverhältnis von 1:2 bis 1:200 vorliegen, weiter bevorzugt in einem Gewichtsverhältnis von 1:4 bis 1:120, mehr bevorzugt in einem Gewichtsverhältnis 1:4 bis 1:80. Dadurch wird eine ausreichende Löslichkeit des Wirkstoffs in dem Lösungsmittel weitgehend sichergestellt.

Nach einer weiter bevorzugten Ausführungsform des erfindungsgemäßen Verfahrens ist es vorgesehen, dass in der Granulierflüssigkeit Wirkstoff und amphiphile Substanz in einem Gewichtsverhältnis von 40:1 bis 1:200 vorliegen, weiter bevorzugt in einem Gewichtsverhältnis von 30:1 bis 1:120, mehr bevorzugt in einem Gewichtsverhältnis 20:1 bis 1:80. Dadurch kann ebenfalls eine ausreichende Löslichkeit des Wirkstoffs in dem Lösungsmittel weitgehend sichergestellt werden. In diesem Zusammenhang ist es erfindungsgemäß weiterhin bevorzugt, wenn das pulverförmige Material ebenfalls die amphiphile Substanz umfasst, und zwar von 0,5 Gew.-% bis zu 20 Gew.-% und vorzugsweise von 1,0 Gew.-% bis zu 10 Gew.-%.

Gemäß einer weiter bevorzugten Ausführungsform des erfindungsgemäßen Verfahrens ist es vorgesehen, dass das Zusammenführen des Lösungsmittels mit dem Wirkstoff bei einer Temperatur oberhalb von 30 °C erfolgt, weiter bevorzugt bei einer Temperatur von 40 °C bis 80 °C und mehr bevorzugt bei einer Temperatur von 45 °C bis 60 °C. Dadurch wird die Löslichkeit des Wirkstoffs in dem Lösungsmittel weiter verbessert ohne Nachteile im Hinblick auf die Stabilität des Wirkstoffs hinnehmen zu müssen.

Nach einer weiter bevorzugten Ausführungsform des erfindungsgemäßen Verfahrens ist es vorgesehen, dass das Verfahren ferner umfasst den Schritt des
d) Trocknens des Granulats.

Entsprechend einer weiter bevorzugten Ausführungsform des erfindungsgemäßen Verfahrens ist es vorgesehen, dass das Verfahren ferner umfasst den Schritt des
e) Verarbeitens des Granulates zu einer Darreichungsform.

Der Schritt des Verarbeitens des Granulates zu einer Darreichungsform kann erfindungsgemäß bevorzugt erfolgen, indem das Granulat zusammen mit oder ohne weitere pharmazeutische Hilfsstoffe in eine Kapsel abgefüllt wird oder indem das Granulat zusammen mit oder ohne weitere pharmazeutische Hilfsstoffe zu einer Tablette verpresst wird. Auch kann das Granulat mit oder ohne weitere Hilfsstoffe in sogenannte Sachets abgefüllt werden.

Gemäß einer weiter bevorzugten Ausführungsform des erfindungsgemäßen Verfahrens ist es vorgesehen, dass die Darreichungsform frei von Natriumdodecylsulfat ist.

Nach einer weiter bevorzugten Ausführungsform des erfindungsgemäßen Verfahrens ist es vorgesehen, dass die Darreichungsform eine unverzögert freisetzende Darreichungsform ist.

Ein typisches erfindungsgemäßes Verfahren umfasst die Schritte des
a) Bereitstellens eines wässrigen Lösungsmittels, umfassend eine amphiphile Substanz, ausgewählt aus der Gruppe bestehend aus einem Polyvinylcaprolactam-polyvinylacetatpolyethylenglykol-pfropfcopolymer oder einem Lauroylmacrogol-32-glycerid;
b) Zusammenführens des Lösungsmittels mit dem Wirkstoff Apixaban unter Erhalt einer Granulierflüssigkeit;
c) Feuchtgranulierens eines pulverförmigen Materials mittels der Granulierflüssigkeit; und optional
d) Trocknens des Granulats; und optional
e) Verarbeitens des Granulates zu einer Darreichungsform.

Im Zusammenhang mit dem vorgenannten typischen erfindungsgemäßen Verfahren wird unter einem wässrigen Lösungsmittel ein Lösungsmittel verstanden, dessen Flüssigkeitskomponente (ohne amphiphile Substanz) zumindest zu 50 Gew.-% aus Wasser besteht, bevorzugt zumindest zu 70 Gew.-% aus Wasser, mehr bevorzugt zumindest zu 90 Gew.-% aus Wasser und noch mehr bevorzugt zumindest zu 95 Gew.-% aus Wasser. In diesem Zusammenhang ist es erfindungsgemäß besonders bevorzugt, wenn die Flüssigkeitskomponente zumindest zu 99 Gew.-% aus Wasser besteht und noch weiter bevorzugt zu 100 Gew.-% aus Wasser.

Neben Wasser kann die Flüssigkeitskomponente ferner einen gewissen Anteil von einer oder mehreren mit Wasser mischbaren flüssigen organischen Flüssigkeitskomponenten enthalten. Als Beispiele für derartige organische Flüssigkeitskomponenten seien DMSO, DMF, Ethanol und Isopropanol genannt.

Die vorliegende Erfindung betrifft ferner ein Granulat erhältlich nach dem erfindungsgemäßen Verfahren.

Die vorliegende Erfindung betrifft ferner eine Darreichungsform erhältlich nach dem erfindungsgemäßen Verfahren oder enthaltend ein erfindungsgemäßes Granulat.

Die nachfolgenden Ausführungsbeispiele dienen der Erläuterung der Erfindung.

### Beispiel 1 (nicht erfindungsgemäß):

Es wurden Filmtabletten als Darreichungsform mit in Tabelle 1 angegebener Formulierung mittels des Granulats (Phase I) hergestellt, das unter Anwendung des unten stehenden Verfahrens gefertigt wurde.

**Tabelle 1:**

| **Bestandteil** | |
|---|---|
| **Phase I** | |
| Cellactose (zusammengesetzt aus 25 Gew.-% Pulvercellulose und 75 Gew.-% Lactose-Monohydrat) | 63,90 mg |
| Crosscarmellose Natrium | 4,00 mg |
| Apixaban (freie Base) | 2,50 mg |
| Dimethylsulfoxid (DMSO, im Endprodukt nicht mehr enthalten) | q.s. |

| **Phase II** | |
|---|---|
| Crosscarmellose Natrium | 4,00 mg |
| Siliciumdioxid | 4,80 mg |
| Magnesiumstearat | 0,80 mg |
| **Endgewicht Tablette** | 80,00 mg |

| **Filmüberzug** | |
|---|---|
| Opadry^{®} (farbig; zusammengesetzt aus Hypromellose, Macrogol, Titandioxid, Eisenoxid, Wasser dem. q.s. (im Endprodukt nicht mehr enthalten)) | 2,50 mg |
| **Endgewicht Filmtablette** | 82,50 mg |

Die Herstellung der Filmtabletten erfolgte, indem zunächst der Füllstoff Cellactose und das Sprengmittel Crosscarmellose Natrium (beide Substanzen bilden das pulverförmige Material) ausgiebig miteinander vermischt wurden. Das so erhaltene Gemisch wurde mittels einer Lösung von Apixaban in DMSO (5 mg/ml) als Granulierflüssigkeit feuchtgranuliert unter Erhalt des erfindungsgemäßen Granulats (Phase I). Das so erhaltene Granulat wurde getrocknet, gesiebt, mit dem Sprengmittel Crosscarmellose Natrium, dem Fließmittel Siliciumdioxid und dem Schmiermittel Magnesiumstearat ausgiebig vermischt und das resultierende Gemisch mittels einer konventionellen Tablettenpresse zu runden flachen biplanen Tablettenkernen verpresst. Die so hergestellten Tablettenkerne wurden mit einem Filmüberzug versehen.

### Beispiel 2 (nicht erfindungsgemäß):

Es wurden Filmtabletten als Darreichungsform mit in Tabelle 2 angegebener Formulierung mittels des Granulats (Phase I) hergestellt, das unter Anwendung des Verfahrens gefertigt wurde.

**Tabelle 2:**

| **Bestandteil** | |
|---|---|
| **Phase I** | |
| Cellactose (zusammengesetzt aus 25 Gew.-% Pulvercellulose und 75 Gew.-% Lactose-Monohydrat) | 63,90 mg |
| Crosscarmellose Natrium | 4,00 mg |
| Apixaban (freie Base) | 2,50 mg |
| Gemisch von DMSO und Wasser im Gewichtsverhältnis 1:1 (DMSO und Wasser im Endprodukt nicht mehr enthalten) | q.s. |

| **Phase II** | |
|---|---|
| Crosscarmellose Natrium | 4,00 mg |
| Siliciumdioxid | 4,80 mg |
| Magnesiumstearat | 0,80 mg |
| **Endgewicht Tablette** | 80,00 mg |
| **Filmüberzug** | |
| Opadry^{®} (farbig; zusammengesetzt aus Hypromellose, Macrogol, Titandioxid, Eisenoxid, Wasser dem. q.s. (im Endprodukt nicht mehr enthalten)) | 2,50 mg |
| **Endgewicht Filmtablette** | 82,50 mg |

Die Herstellung der Filmtabletten erfolgte analog dem Beispiel 1, wobei das pulverförmige Gemisch mittels einer Lösung von Apixaban in DMSO/Wasser (0,5 mg/ml) als Granulierflüssigkeit feuchtgranuliert wurde.

### Beispiel 3 (nicht erfindungsgemäß):

Es wurden Filmtabletten als Darreichungsform mit in Tabelle 3 angegebener Formulierung mittels des Granulats (Phase I) hergestellt, das unter Anwendung des Verfahrens gefertigt wurde.

**Tabelle 3:**

| **Bestandteil** | |
|---|---|
| **Phase I** | |
| Cellactose (zusammengesetzt aus 25 Gew.-% Pulvercellulose und 75 Gew.-% Lactose-Monohydrat) | 63,90 mg |
| Crosscarmellose Natrium | 4,00 mg |
| Apixaban (freie Base) | 2,50 mg |
| Dimethylformamid (DMF, im Endprodukt nicht mehr | q.s. |
| enthalten) | |

| **Phase II** | |
|---|---|
| Crosscarmellose Natrium | 4,00 mg |
| Siliciumdioxid | 4,80 mg |
| Magnesiumstearat | 0,80 mg |
| **Endgewicht Tablette** | 80,00 mg |
| **Filmüberzug** | |
| Opadry^{®} (farbig; zusammengesetzt aus Hypromellose, Macrogol, Titandioxid, Eisenoxid, Wasser dem. q.s. (im Endprodukt nicht mehr enthalten)) | 2,50 mg |
| **Endgewicht Filmtablette** | 82,50 mg |

Die Herstellung der Filmtabletten erfolgte analog dem Beispiel 1, wobei das pulverförmige Gemisch mittels einer Lösung von Apixaban in DMF (3,0 mg/ml) als Granulierflüssigkeit feuchtgranuliert wurde.

### Beispiel 4 (nicht erfindungsgemäß):

Es wurden Filmtabletten als Darreichungsform mit in Tabelle 4 angegebener Formulierung mittels des Granulats (Phase I) hergestellt, das unter Anwendung des Verfahrens gefertigt wurde.

**Tabelle 4:**

| **Bestandteil** | |
|---|---|
| **Phase I** | |
| Cellactose (zusammengesetzt aus 25 Gew.-% Pulvercellulose und 75 Gew.-% Lactose-Monohydrat) | 63,90 mg |
| Crosscarmellose Natrium | 4,00 mg |
| Apixaban (freie Base) | 2,50 mg |
| Gemisch von DMF und Wasser im Gewichtsverhältnis 1:1 (DMF und Wasser im Endprodukt nicht mehr enthalten) | q.s. |

| **Phase II** | |
|---|---|
| Crosscarmellose Natrium | 4,00 mg |
| Siliciumdioxid | 4,80 mg |
| Magnesiumstearat | 0,80 mg |
| **Endgewicht Tablette** | 80,00 mg |
| **Filmüberzug** | |
| Opadry^{®} (farbig; zusammengesetzt aus Hypromellose, Macrogol, Titandioxid, Eisenoxid, Wasser dem. q.s.(im Endprodukt nicht mehr enthalten)) | 2,50 mg |
| **Endgewicht Filmtablette** | 82,50 mg |

Die Herstellung der Filmtabletten erfolgte analog dem Beispiel 1, wobei das pulverförmige Gemisch mittels einer Lösung von Apixaban in DMF/Wasser (0,5 mg/ml) als Granulierflüssigkeit feuchtgranuliert wurde.

### Ausführungsbeispiel 5 (erfindungsgemäß):

Es wurden Filmtabletten als erfindungsgemäße Darreichungsform mit in Tabelle 5 angegebener Formulierung mittels des erfindungsgemäßen Granulats (Phase I) hergestellt, das unter Anwendung des erfindungsgemäßen Verfahrens gefertigt wurde.

**Tabelle 5:**

| **Bestandteil** | |
|---|---|
| **Phase I** | |
| Cellactose (zusammengesetzt aus 25 Gew.-% Pulvercellulose und 75 Gew.-% Lactose-Monohydrat) | 33,90 mg |
| Crosscarmellose Natrium | 4,00 mg |
| Apixaban (freie Base) | 2,50 mg |
| Soluplus^{®} (BASF, Polyvinylcaprolactampolyvinylacetat-polyethylenglykolpfropfcopolymer mit einem mittleren | 200,00 mg |
| Molekulargewicht von 90000 bis 140000 g/mol) | |
| Wasser (im Endprodukt nicht mehr enthalten) | q.s. |

| **Phase II** | |
|---|---|
| Crosscarmellose Natrium | 4,00 mg |
| Siliciumdioxid | 4,80 mg |
| Magnesiumstearat | 0,80 mg |
| **Endgewicht Tablette** | 250,00 mg |
| **Filmüberzug** | |
| Opadry^{®} (farbig; zusammengesetzt aus Hypromellose, Macrogol, Titandioxid, Eisenoxid, Wasser dem. q.s. (im Endprodukt nicht mehr enthalten)) | 5,00 mg |
| **Endgewicht Filmtablette** | 255,00 mg |

Die Herstellung der Filmtabletten erfolgte analog dem Beispiel 1, wobei das pulverförmige Gemisch mittels einer Lösung/Dispersion von Apixaban in Wasser/Soluplus^{®} (2,5 mg/ml) als Granulierflüssigkeit feuchtgranuliert wurde.

Besagte Granulierflüssigkeit wurde hergestellt, in dem das Lösungsmittel, d.h. eine 20 Gew.-%ige Lösung von Soluplus^{®} als amphiphile Substanz in Wasser, auf eine Temperatur von 50 °C erwärmt und dazu 2,5 mg Apixaban pro ml Lösungsmittel gegeben wurden. Nach Einwirkung eines Ultra-Turrax^{®} auf die Granulierflüssigkeit über einen Zeitraum von 4 h waren ca. 54 % des eingesetzten Apixabans in dem Lösungsmittel gelöst und der Rest in dem Lösungsmittel dispergiert enthalten.

### Ausführungsbeispiel 6 (erfindungsgemäß):

Es wurden Filmtabletten als erfindungsgemäße Darreichungsform mit in Tabelle 6 angegebener Formulierung mittels des erfindungsgemäßen Granulats (Phase I) hergestellt, das unter Anwendung des erfindungsgemäßen Verfahrens gefertigt wurde.

**Tabelle 6:**

| **Bestandteil** | |
|---|---|
| **Phase I** | |
| Cellactose (zusammengesetzt aus 25 Gew.-% Pulvercellulose und 75 Gew.-% Lactose-Monohydrat) | 33,90 mg |
| Crosscarmellose Natrium | 4,00 mg |
| Apixaban (freie Base) | 2,50 mg |
| Gelucire^{®} 44/14 (Gattefossé, Gelucire^{®} 44/14 ist eine Mischung von PEG-Estern (d.h. Lauroyl-macrogol-32-glycerid), einer kleinen Glycerid-Fraktion und freiem PEG | 100,00 mg |
| Wasser (im Endprodukt nicht mehr enthalten) | q.s. |

| **Phase II** | |
|---|---|
| Crosscarmellose Natrium | 4,00 mg |
| Siliciumdioxid | 4,80 mg |
| Magnesiumstearat | 0,80 mg |
| **Endgewicht Tablette** | 150,00 mg |

| **Filmüberzug** | |
|---|---|
| Opadry^{®} (farbig; zusammengesetzt aus Hypromellose, Macrogol, Titandioxid, Eisenoxid, Wasser dem. q.s.(im Endprodukt nicht mehr enthalten)) | 3,00 mg |
| **Endgewicht Filmtablette** | 153,00 mg |

Die Herstellung der Filmtabletten erfolgte analog dem Beispiel 1, wobei das pulverförmige Gemisch mittels einer Lösung/Dispersion von Apixaban in Wasser/Gelucire^{®} (2,5 mg/ml) als Granulierflüssigkeit feuchtgranuliert wurde.

Besagte Granulierflüssigkeit wurde hergestellt, in dem das Lösungsmittel, d.h. eine 10 Gew.-%ige Lösung von Gelucire^{®} als amphiphile Substanz in Wasser, auf eine Temperatur von 50 °C erwärmt und dazu 2,5 mg Apixaban pro ml Lösungsmittel gegeben wurden.

### Ausführungsbeispiel 7 (erfindungsgemäß):

Es wurden Filmtabletten als erfindungsgemäße Darreichungsform mit in Tabelle 7 angegebener Formulierung mittels des erfindungsgemäßen Granulats (Phase I) hergestellt, das unter Anwendung des erfindungsgemäßen Verfahrens gefertigt wurde.

**Tabelle 7:**

| **Bestandteil** | |
|---|---|
| **Phase I** | |
| mikrokristalline Cellulose | 49,00 mg |
| Na-Carboxymethylstärke | 3,00 mg |
| Apixaban (freie Base) | 2,50 mg |
| Soluplus^{®} (BASF, Polyvinylcaprolactampolyvinylacetat-polyethylenglykolpfropfcopolymer mit einem mittleren Molekulargewicht von 90000 bis 140000 g/mol) | 20,00 mg |
| Wasser (im Endprodukt nicht mehr enthalten) | q.s. |

| **Phase II** | |
|---|---|
| mikrokristalline Cellulose | 22,50 mg |
| Siliciumdioxid | 2,00 mg |
| Magnesiumstearat | 1,00 mg |
| **Endgewicht Tablette** | 100,00 mg |

| **Filmüberzug** | |
|---|---|
| Opadry^{®} (farbig; zusammengesetzt aus Hypromellose, Macrogol, Titandioxid, Eisenoxid, Wasser dem. q.s.(im Endprodukt nicht mehr enthalten)) | 5,00 mg |
| **Endgewicht Filmtablette** | 105,00 mg |

Die Herstellung der Filmtabletten erfolgte analog dem Beispiel 1, wobei das pulverförmige Gemisch mittels einer Lösung/Dispersion von Apixaban in Wasser/Soluplus^{®} (2,5 mg/ml) als Granulierflüssigkeit feuchtgranuliert wurde.

Besagte Granulierflüssigkeit wurde hergestellt, in dem das Lösungsmittel, d.h. eine 2 Gew.-%ige Lösung von Soluplus^{®} als amphiphile Substanz in Wasser, auf eine Temperatur von 50 °C erwärmt und dazu 2,5 mg Apixaban pro ml Lösungsmittel gegeben wurden. Nach Einwirkung eines Ultra-Turrax^{®} auf die Granulierflüssigkeit über einen Zeitraum von 4 h war ein Teil des eingesetzten Apixabans in dem Lösungsmittel gelöst und der Rest in dem Lösungsmittel dispergiert enthalten.

### Ausführungsbeispiel 8 (erfindungsgemäß):

Es wurden Filmtabletten als erfindungsgemäße Darreichungsform mit in Tabelle 8 angegebener Formulierung mittels des erfindungsgemäßen Granulats (Phase I) hergestellt, das unter Anwendung des erfindungsgemäßen Verfahrens gefertigt wurde.

**Tabelle 8:**

| **Bestandteil** | |
|---|---|
| **Phase I** | |
| mikrokristalline Cellulose | 59,00 mg |
| Na-Carboxymethylstärke | 3,00 mg |
| Apixaban (freie Base) | 2,50 mg |
| Gelucire^{®} 44/14 (Gattefossé, Gelucire^{®} 44/14 ist eine Mischung von PEG-Estern (d.h. Lauroyl-macrogol-32-glycerid), einer kleinen Glycerid-Fraktion und freiem PEG | 10,00 mg |
| Wasser (im Endprodukt nicht mehr enthalten) | q.s. |

| **Phase II** | |
|---|---|
| mikrokristalline Cellulose | 22,50 mg |
| Siliciumdioxid | 2,00 mg |
| Magnesiumstearat | 1,00 mg |
| **Endgewicht Tablette** | 100,00 mg |

| **Filmüberzug** | |
|---|---|
| Opadry^{®} (farbig; zusammengesetzt aus Hypromellose, Macrogol, Titandioxid, Eisenoxid, Wasser dem. q.s. (im Endprodukt nicht mehr enthalten)) | 5,00 mg |
| **Endgewicht Filmtablette** | 105,00 mg |

Die Herstellung der Filmtabletten erfolgte analog dem Beispiel 1, wobei das pulverförmige Gemisch mittels einer Lösung/Dispersion von Apixaban in Wasser/Gelucire^{®} (2,5 mg/ml) als Granulierflüssigkeit feuchtgranuliert wurde.

Besagte Granulierflüssigkeit wurde hergestellt, in dem das Lösungsmittel, d.h. eine 1 Gew.-%ige Lösung von Gelucire^{®} als amphiphile Substanz in Wasser, auf eine Temperatur von 50 °C erwärmt und dazu 2,5 mg Apixaban pro ml Lösungsmittel gegeben wurden.

### Ausführungsbeispiel 9 (erfindungsgemäß):

Es wurden Tabletten als erfindungsgemäße Darreichungsform mit in Tabelle 9 angegebener Formulierung mittels des erfindungsgemäßen Granulats (Phase I) hergestellt, das unter Anwendung des erfindungsgemäßen Verfahrens gefertigt wurde.

**Tabelle 9:**

| **Bestandteil** | |
|---|---|
| **Phase I** | |
| Mikrokristalline Cellulose (Avicel PH 101) | 64,00 mg |
| Na-Carboxymethylstärke (Primojel) | 3,00 mg |
| Apixaban (freie Base) | 2,50 mg |
| Povidon 30 (Kollidon K-30) | 1,50 |
| Soluplus^{®} (BASF, Polyvinylcaprolactampolyvinylacetat-polyethylenglykolpfropfcopolymer mit einem mittleren | 5,00 mg |
| Molekulargewicht von 90000 bis 140000 g/mol) | |
| Wasser (im Endprodukt nicht mehr enthalten) | 45,00 |

| **Phase II** | |
|---|---|
| Mikrokristalline Cellulose (Avicel PH 102) | 21,00 mg |
| Siliciumdioxid, hochdispers (Aerosil 200) | 2,00 mg |
| Magnesiumstearat | 1,00 mg |
| **Endgewicht Tablette** (ohne Film) | 100,00 mg |

Die Herstellung der Tabletten erfolgte analog dem Beispiel 1, wobei das pulverförmige Gemisch mittels einer Lösung/Dispersion von Apixaban in Wasser/Soluplus^{®} als Granulierflüssigkeit feuchtgranuliert wurde.

Besagte Granulierflüssigkeit wurde hergestellt, indem das Soluplus^{®} als amphiphile Substanz unter Einwirkung eines Ultra-Turrax^{®} zunächst in Wasser gelöst und in der resultierenden Lösung Apixaban dann unter Einwirkung eines Ultra-Turrax^{®} fein verteilt wurde. Die so erhaltene Suspension wurde über Nacht bei 50 °C mit einem Magnetrührer gerührt.

### Ausführungsbeispiel 10 (erfindungsgemäß) :

Es wurden Tabletten als erfindungsgemäße Darreichungsform mit in Tabelle 10 angegebener Formulierung mittels des erfindungsgemäßen Granulats (Phase I) hergestellt, das unter Anwendung des erfindungsgemäßen Verfahrens gefertigt wurde.

**Tabelle 10:**

| **Bestandteil** | |
|---|---|
| **Phase I** | |
| Mikrokristalline Cellulose (Avicel PH 101) | 128,00 mg |
| Na-Carboxymethylstärke (Primojel) | 6,00 mg |
| Apixaban (freie Base) | 5,00 mg |
| Povidon 30 (Kollidon K-30) | 3,00 |
| Soluplus^{®} (BASF, Polyvinylcaprolactam- | 10,00 mg |
| polyvinylacetat-polyethylenglykolpfropfcopolymer mit einem mittleren Molekulargewicht von 90000 bis 140000 g/mol) | |
| Wasser (im Endprodukt nicht mehr enthalten) | 90,00 |

| **Phase II** | |
|---|---|
| Mikrokristalline Cellulose (Avicel PH 102) | 42,00 mg |
| Siliciumdioxid, hochdispers (Aerosil 200) | 4,00 mg |
| Magnesiumstearat | 2,00 mg |
| **Endgewicht Tablette** (ohne Film) | 200,00 mg |

Die Herstellung der Tabletten erfolgte analog dem Beispiel 1, wobei das pulverförmige Gemisch mittels einer Lösung/Dispersion von Apixaban in Wasser/Soluplus^{®} als Granulierflüssigkeit feuchtgranuliert wurde.

Besagte Granulierflüssigkeit wurde hergestellt, indem das Soluplus^{®} als amphiphile Substanz unter Einwirkung eines Ultra-Turrax^{®} zunächst in Wasser gelöst und in der resultierenden Lösung Apixaban dann unter Einwirkung eines Ultra-Turrax^{®} fein verteilt wurde. Die so erhaltene Suspension wurde über Nacht bei 50 °C mit einem Magnetrührer gerührt.

### Ausführungsbeispiel 11 (erfindungsgemäß):

Es wurden Tabletten als erfindungsgemäße Darreichungsform mit in Tabelle 11 angegebener Formulierung mittels des erfindungsgemäßen Granulats (Phase I) hergestellt, das unter Anwendung des erfindungsgemäßen Verfahrens gefertigt wurde.

**Tabelle 11:**

| **Bestandteil** | |
|---|---|
| **Phase I** | |
| Mikrokristalline Cellulose (Avicel PH 101) | 87,87 mg |
| Croscarmellose | 4,00 mg |
| Apixaban (freie Base) | 2,50 mg |
| Soluplus^{®} (BASF, Polyvinylcaprolactampolyvinylacetat-polyethylenglykolpfropfcopolymer mit einem mittleren Molekulargewicht von 90000 bis 140000 g/mol) | 0,13 mg |
| Soluplus^{®} (BASF, Polyvinylcaprolactampolyvinylacetat-polyethylenglykolpfropfcopolymer mit einem mittleren Molekulargewicht von 90000 bis 140000 g/mol) | 5,00 mg |
| Wasser (im Endprodukt nicht mehr enthalten) | 45,00 |

| **Phase II** | |
|---|---|
| Magnesiumstearat | 0,50 mg |
| **Endgewicht Tablette** (ohne Film) | 100,00 mg |

Die Herstellung der Tabletten erfolgte analog dem Beispiel 1, wobei das pulverförmige Gemisch mittels einer Lösung/Dispersion von Apixaban in Wasser/0,13 mg Soluplus^{®} als Granulierflüssigkeit feuchtgranuliert wurde.

Besagte Granulierflüssigkeit wurde hergestellt, indem das Soluplus^{®} als amphiphile Substanz unter Einwirkung eines Ultra-Turrax^{®} zunächst in Wasser gelöst und in der resultierenden Lösung Apixaban dann unter Einwirkung eines Ultra-Turrax^{®} fein verteilt wurde. Die so erhaltene Suspension wurde über Nacht bei 50 °C mit einem Magnetrührer gerührt.

### Ausführungsbeispiel 12 (erfindungsgemäß) :

Es wurden Tabletten als erfindungsgemäße Darreichungsform mit in Tabelle 12 angegebener Formulierung mittels des erfindungsgemäßen Granulats (Phase I) hergestellt, das unter Anwendung des erfindungsgemäßen Verfahrens gefertigt wurde.

**Tabelle 12:**

| **Bestandteil** | |
|---|---|
| **Phase I** | |
| Mikrokristalline Cellulose (Avicel PH 101) | 175,74 mg |
| Croscarmellose | 8,00 mg |
| Apixaban (freie Base) | 5,00 mg |
| Soluplus^{®} (BASF, Polyvinylcaprolactampolyvinylacetat-polyethylenglykolpfropfcopolymer mit einem mittleren Molekulargewicht von 90000 bis 140000 g/mol) | 0,26 mg |
| Soluplus^{®} (BASF, Polyvinylcaprolactampolyvinylacetat-polyethylenglykolpfropfcopolymer mit einem mittleren Molekulargewicht von 90000 bis 140000 g/mol) | 10,00 mg |
| Wasser (im Endprodukt nicht mehr enthalten) | 90,00 |

| **Phase II** | |
|---|---|
| Magnesiumstearat | 1,00 mg |
| **Endgewicht Tablette** (ohne Film) | 200,00 mg |

Die Herstellung der Tabletten erfolgte analog dem Beispiel 1, wobei das pulverförmige Gemisch mittels einer Lösung/Dispersion von Apixaban in Wasser/0,26 mg Soluplus^{®} als Granulierflüssigkeit feuchtgranuliert wurde.

Besagte Granulierflüssigkeit wurde hergestellt, indem das Soluplus^{®} als amphiphile Substanz unter Einwirkung eines Ultra-Turrax^{®} zunächst in Wasser gelöst und in der resultierenden Lösung Apixaban dann unter Einwirkung eines Ultra-Turrax^{®} fein verteilt wurde. Die so erhaltene Suspension wurde über Nacht bei 50 °C mit einem Magnetrührer gerührt.

## Patentansprüche

1. Verfahren zur Herstellung eines pharmazeutischen Granulats, enthaltend als Wirkstoff Apixaban oder ein Salz davon, umfassend die Schritte des
a) Bereitstellens eines Lösungsmittels;
b) Zusammenführens des Lösungsmittels mit dem Wirkstoff unter Erhalt einer Granulierflüssigkeit;
c) Feuchtgranulierens eines pulverförmigen Materials mittels der Granulierflüssigkeit,
wobei in dem Lösungsmittel eine amphiphile Substanz enthalten ist und die amphiphile Substanz ein Polyvinylcaprolactampolyvinylacetat-polyethylenglykol- pfropfcopolymer ist oder ausgewählt ist aus der Gruppe bestehend aus Caprylocaproylmacrogol-8-glycerid, Lauroyl-macrogol-32-glycerid, Stearoylmacrogol-32-glycerid, Oleoyl-macrogol-6-glycerid, Linoleoylmacrogol-6-glycerid und Lauroyl-macrogol-6-glycerid, und der Wirkstoff in dem Lösungsmittel gelöst, gelöst und dispergiert, gelöst und suspendiert oder gelöst, dispergiert und suspendiert enthalten ist.

2. Verfahren nach Anspruch 1, **dadurch gekennzeichnet, dass** das Lösungsmittel Wasser umfasst.

3. Verfahren nach einem der voranstehenden Ansprüche, **dadurch gekennzeichnet, dass** der Wirkstoff Apixaban ist.

4. Verfahren nach einem der Ansprüche 1 bis 3, **dadurch gekennzeichnet, dass** in der Granulierflüssigkeit Wirkstoff und Lösungsmittel in einem Gewichtsverhältnis von 1:100 bis 1:2500 vorliegen, bevorzugt in einem Gewichtsverhältnis von 1:200 bis 1:1000, mehr bevorzugt in einem Gewichtsverhältnis von 1:200 bis 1:600.

5. Verfahren nach einem der Ansprüche 1 bis 4, **dadurch gekennzeichnet, dass** in der Granulierflüssigkeit Wirkstoff und amphiphile Substanz in einem Gewichtsverhältnis von 1:2 bis 1:200 vorliegen, bevorzugt in einem Gewichtsverhältnis von 1:4 bis 1:120, mehr bevorzugt in einem Gewichtsverhältnis 1:4 bis 1:80.

6. Verfahren nach einem der voranstehenden Ansprüche, **dadurch gekennzeichnet, dass** das Zusammenführen des Lösungsmittels mit dem Wirkstoff bei einer Temperatur oberhalb von 30 °C erfolgt.

7. Verfahren nach einem der voranstehenden Ansprüche, **dadurch gekennzeichnet, dass** das Verfahren ferner umfasst den Schritt des
d) Trocknens des Granulats.

8. Granulat, erhältlich nach einem Verfahren gemäß einem der Ansprüche 1 bis 7.

9. Darreichungsform, enthaltend ein Granulat gemäß Anspruch 8.

## Claims

1. Method for producing a pharmaceutical granulate comprising as active substance apixaban or a salt thereof, comprising the steps of
a) providing a solvent;
b) combining the solvent with the active substance to obtain a granulating liquid;
c) wet granulating a pulverulent material using the granulating liquid,
wherein an amphiphilic substance is present in the solvent and the amphiphilic substance is a polyvinylcaprolactam-polyvinyl acetate-polyethylene glycol graft copolymer or is selected from the group consisting of caprylocaproyl macrogol-8 glyceride, lauroyl macrogol-32 glyceride, stearoyl macrogol-32 glyceride, oleoyl macrogol-6 glyceride, linoleoyl macrogol-6 glyceride and lauroyl macrogol-6 glyceride, and the active substance is present in the solvent in dissolved form, in dissolved and dispersed form, in dissolved and suspended form, or in dissolved, dispersed and suspended form.

2. Method according to Claim 1, **characterized in that** the solvent comprises water.

3. Method according to either of the preceding claims, **characterized in that** the active substance is apixaban.

4. Method according to any of Claims 1 to 3, **characterized in that** active substance and solvent are present in the granulating liquid in a weight ratio of 1:100 to 1:2500, preferably in a weight ratio of 1:200 to 1:1000, more preferably in a weight ratio of 1:200 to 1:600.

5. Method according to any of Claims 1 to 4, **characterized in that** active substance and amphiphilic substance are present in the granulating liquid in a weight ratio of 1:2 to 1:200, preferably in a weight ratio of 1:4 to 1:120, more preferably in a weight ratio of 1:4 to 1:80.

6. Method according to any of the preceding claims, **characterized in that** the solvent is combined with the active substance at a temperature above 30°C.

7. Method according to any of the preceding claims, **characterized in that** the method further comprises the step of
d) drying the granulate.

8. Granulate obtainable by a method according to any of Claims 1 to 7.

9. Dosage form comprising a granulate according to Claim 8.

## Revendications

1. Procédé de préparation de granulés pharmaceutiques contenant comme principe actif de l'apixaban ou l'un de ses sels, comprenant les étapes consistant à :
a) fournir un solvant ;
b) combiner le solvant avec la substance active afin d'obtenir un liquide de granulation ;
c) granuler par voie humide un matériau pulvérulent à l'aide du liquide de granulation,
dans lequel le solvant contient une substance amphiphile, et la substance amphiphile est un copolymère greffé de polyvinylcaprolactamepolyvinylacétate-polyéthylèneglycol ou est choisie dans le groupe constitué de caprylocaproyl-macrogol-8-glycéride, lauroyl-macrogol-32-glycéride, stéaroyl-macrogol-32-glycéride, oléoyl-macrogol-6-glycéride, linoléoylmacrogol-6-glycéride et lauroyl-macrogol-6-glycéride, et le principe actif se trouve dans le solvant en étant dissous, dissous et dispersé, dissous et mis en suspension, ou bien dissous, dispersé et mis en suspension.

2. Procédé selon la revendication 1, **caractérisé en ce que** le solvant comprend de l'eau.

3. Procédé selon l'une des revendications précédentes, **caractérisé en ce que** le principe actif est l'apixaban.

4. Procédé selon l'une des revendications 1 à 3, **caractérisé en ce que** la substance active et le solvant sont présents dans le liquide de granulation selon un rapport pondéral compris entre 1:100 et 1:2500, de préférence selon un rapport pondéral compris entre 1:200 et 1:1000, de manière plus préférée selon un rapport pondéral compris entre 1:200 et 1:600.

5. Procédé selon l'une des revendications 1 à 4, **caractérisé en ce que** la substance active et la substance amphiphile sont présentes dans le liquide de granulation selon un rapport pondéral compris entre 1:2 et 1:200, de préférence selon un rapport pondéral compris entre 1:4 et 1:120, de manière plus préférée selon un rapport pondéral compris entre 1:4 et 1:80.

6. Procédé selon l'une des revendications précédentes, **caractérisé en ce que** la combinaison du solvant avec le principe actif intervient à une température supérieure à 30 °C.

7. Procédé selon l'une des revendications précédentes, **caractérisé en ce que** le procédé comprend en outre l'étape consistant à d) sécher les granulés.

8. Granulés pouvant être obtenus par un procédé selon l'une quelconque des revendications 1 à 7.

9. Forme galénique contenant des granulés selon la revendication 8.
